Europäisches Patentamt

European Patent Office

Office européen des brevets

(11) Veröffentlichungsnummer: **0 312 650 B1**

## EUROPÄISCHE PATENTSCHRIFT

(45) Veröffentlichungstag der Patentschrift: 08.01.92

(51) Int. Cl.⁵: **B23K  26/00**, B23K 26/04, A61B 17/36

(21) Anmeldenummer: 87119372.8

(22) Anmeldetag: 30.12.87

(54) **Verfahren und Vorrichtung zur Materialbearbeitung mit Hilfe eines Lasers.**

(30) Priorität: 03.10.87 DE 3733489

(43) Veröffentlichungstag der Anmeldung: 26.04.89 Patentblatt  89/17

(45) Bekanntmachung des Hinweises auf die Patenterteilung: 08.01.92 Patentblatt  92/02

(84) Benannte Vertragsstaaten: AT BE CH DE ES FR GB GR IT LI NL SE

(56) Entgegenhaltungen:
EP-A- 0 195 375
DE-A- 3 245 846
US-A- 4 443 684
US-A- 4 504 727

APPLIED PHYSICS B, Band B42, Nr. 2, Februar 1987, Seiten 73-78, Springer-Verlag, Heidelberg, DE; P. TENG et al.: "Optical studies of pulsed-laser fragmentation of biliary calculi"

(73) Patentinhaber: Telemit Electronic GmbH
Heidemannstrasse 17
W-8000 München 45(DE)

(72) Erfinder: Meyer, Wilhelm, Dipl.-Ing.
Röntgenstrasse 19
W-2400 Lübeck 1(DE)
Erfinder: Engelhardt, Ralf, Dipl.-Phys.
Fischergrube 40
W-2400 Lübeck 1(DE)

(74) Vertreter: Feldkamp, Rainer, Dipl.-Ing. Patentanwälte Wallach, Koch, Dr. Haibach, Feldkamp et al
P.O. Box 121120
W-8000 München 12(DE)

## Beschreibung

Die Erfindung bezieht sich auf ein Verfahren sowie auf eine Vorrichtung zur Materialbearbeitung mit Hilfe eines Lasers der im Oberbegriff der Ansprüche 1,2 bzw. 7,10 genannten Art.

Bei einem bekannten Verfahren dieser Art (Optical Studies of Pulsed-Laser Fragmentation of Biliary Calculi, Applied Physics B, Springer Verlag 1987 Seiten 73-78) wird das Ausgangssignal eines Lasers über eine Laseroptik und einen Lichtwellenleiter auf Harn- oder Gallensteine gerichtet, um diese zu zertrümmern. In der Laseroptik ist ein halbdurchlässiger Spiegel angeordnet, der einen Teil des von dem Stein reemitierten, zurückreflektierten oder zurückgestreuten Lichtes, das über den Lichtwellenleiter zurückgeleitet wird, auf einen Detektor (1) leitet, dem eine Auswerteschaltung in Form eines Spektralanalysators nachgesschaltet ist.

Hierbei ist es sehr schwierig, den Lichtwellenleiter so zu führen, daß eine Beeinträchtigung des den Stein umgebenden Gewebes vollständig ausgeschlossen wird. Auch eine visuelle Kontrolle oder eine Kontrolle mit Hilfe radiologischer Verfahren kann nicht zuverlässig ausschließen, daß die Laserenergie auf Gewebeteile trifft.

Aus der EP-A-0 195 375 ist weiterhin ein Verfahren und eine Vorrichtung bekannt, bei dem bzw. bei der das zu bearbeitende Material mit Hilfe eines Pilotlasers geringerer Leistung angestrahlt und das Spektrum des zurückkehrenden Lichtes mit Hilfe eines Korrelationsverfahrens mit einem vorher gespeichertenn Spektrum verglichen wird, um festzustellen, ob das von dem Laserstrahl getroffene Material ein zu bearbeitendes Material ist oder nicht. Zu diesem Zweck wird eine Wellenlänge nach der anderen abgetastet, um das Gesamtspektrum auf einer Zeitachse darzustellen, was sowohl hinsichtlich. der Auswertezeit als auch hinsichtlich der erforderlichen Geräte aufwendig ist. Bei einer weiteren Ausführungsform dieses Verfahrens werden nur die Intensitäten bei wenigen Wellenlängen ermittelt, um die Feststellung des Materials zu ermöglichen. Hierbei wird jedoch ein Vergleich der Intensitäten bei zumindestens zwei verschiedenen Wellenlängen durchgeführt.

Der Erfindung liegt die Aufgabe zugrunde, ein Verfahren sowie eine Vorrichtung der eingangs genannten Art zu schaffen, bei dem bzw bei der bei geingem Aufwand automatisch eine Begrenzung der Bearbeitung auf gewünschte Bereiche bzw. Materialien erreicht wird, so daß beispielsweise im Fall der Zertrümmerung von menschlichen Steinen, wie Harn- und Gallensteinen, eine Schädigung des den Stein einbettenden Gewebes sicher vermieden wird.

Diese Aufgabe wird durch die Merkmale des Patentanspruchs 1,2 bzw. 7,10 gelöst. Vorteilhafte Ausgestaltungen und Weiterbildungen der Erfindung ergeben sich aus den jeweiligen Unteransprüchen.

Durch die erfindungsgemäße Ausgestaltung des Verfahrens bzw. der Vorrichtung ergibt sich eine exakt steuerbare Bearbeitung des zu bearbeitenden Materials, was insbesondere bei Verwendung des Verfahrens bzw. der Vorrichtung zur Zertrümmerung von menschlichen Steinen, beispielsweise Harn- und Gallensteinen wesentlich ist. Hierbei kann der Laser so gesteuert werden, daß sich eine gewebeschonende, das heißt steinselektive Impulsabgabe ergibt. Die Regelung der applizierten Energie kann hierbei über eine Änderung der Leistungsdichte oder durch eine Änderung der Impulsdauer bzw. durch Regelung beider Parameter erfolgen.

Gemäß einer weiteren Ausführungsform wird der Laserimpuls vor Erreichen seiner maximalen Energie unterbrochen, wenn das Ausgangssignal der Detektoranordnung innerhalb einer vorgegebenen Zeit nach Auslösung des Laserimpulses einen vorgegebenen Schwellwert nicht überschreitet. Bei dieser Ausführungsform wird die erfindungsgemäße Feststellung berücksichtigt, daß das Ausgangssignal der Detektoranordnung bei Auftreffen des Laserimpulses auf hartes Material bereits kurz nach Auslösung des Laserimpulses erhebliche Werte annimmt, während ein Anstieg dieses Ausgangssignals bei Auftreffen auf Gewebematerial oder weiches Material erst nach einer erheblich längeren Zeit erfolgt. Wenn daher innerhalb einer vorgegebenen Zeit nach Auslösen des Laserimpulses noch kein Anstieg des Ausgangssignals auftritt, so deutet dies an, daß der Laserimpuls auf relativ weiches Material auftrifft und dann vor Erreichen seines Maximalwertes beispielsweise durch Sperren eines optischen Schalters beendet werden kann.

Das erfindungsgemäße Verfahren bzw. die Vorrichtung ist auch zur Bearbeitung anderer Materialien, wie z. B. eng begrenzter Bereiche von Halbleitermaterialien bei der Herstellung integrierter Schaltungen geeignet.

Eine weitere Anwendung besteht beispielsweise in der Angioplastie.

Bei dem erfindungsgemäßen Verfahren bzw. der Vorrichtung wird der Laser bzw. der diesem nachgeschaltete optische Schalter gemäß einer Ausführungsform so gesteuert, daß zunächst nur Meßimpulse abgegeben werden, deren Impulsenergie so niedrig gehalten wird, daß es noch zu keinem dielektrischen Durchbruch kommt. Hierbei wird der zeitliche Funktionsverlauf des auf die Detektoranordnung einfallenden Lichtes ausgewertet. Wenn diese Auswertung das Vorliegen von zu bearbeitendem Material anzeigt, erfolgt eine Hochsteuerung der Laserenergie, womit gewährleistet ist, daß der dielektrische Durchbruch lediglich an dem zu bearbeitenden Material, beispielsweise dem Stein erfolgt.

Nachfolgend kann dann mit jedem Bearbeitungsimpuls eine Analyse des zeitlichen Funktionsverlaufes des auf den Detektor einfallenden Lichtes erfolgen, wobei sich dieser zeitliche Funktionsverlauf deutlich ändert, wenn der Laserimpuls statt auf das zu bearbeitende Material auf ein Umgebungsmaterial auftrifft Wenn dies festgestellt wird, wird die Laserenergie auf einen geringeren Wert herabgesetzt.

Alternativ ist es möglich, auf jeden Bearbeitungsimpuls mit hoher Energie einen Meßimpuls folgen zu lassen, der feststellt, ob noch zu bearbeitendes Material von dem Laserimpuls getroffen wird. In jedem Fall kann eine Beschädigung des das zu bearbeitende Material umgebenden Materials sicher vermieden werden.

Weiterhin ist es gemäß einer bevorzugten Ausführungsform möglich, mit Hilfe des optischen Schalters einen Laserimpuls vor Erreichen der maximalen Energie zu unterbrechen, wenn der zeitliche Verlauf des Ausgangssignals der Detektoranordnung anzeigt, daß der Laserimpuls nicht auf zu bearbeitendes Material gerichtet ist, so daß eine Schädigung von nicht zu bearbeitendem Material vermieden wird.

Zusätzlich zur optischen Auswertung kann auch das den dielektrischen Durchbruch begleitende akustische Signal erfaßt und ausgewertet werden, um zum Beispiel die Zahl der applizierten Bearbeitungsimpulse zu Dokumentationszwecken festzuhalten.

Die Erfindung wird im folgenden anhand von in der Zeichnung dargestellten Ausführungsbeispielen noch näher erläutert.

In der Zeichnung zeigen:

Fig. 1 eine Ausführungsform einer Vorrichtung zur Durchführung des Verfahrens

Fig. 2-6 Diagramme, die Beispiele für den zeitlichen Funktionsverlauf des auf die Detektoranordnung einfallenden Lichtes zeigen

Fig. 7 idealisierte Darstellungen der Kurvenverläufe nach den Fig. 5 und 6

Fig. 8 eine erste Ausführungsform der Auswerteschaltung

Fig. 9 eine zweite Ausführungsform der Auswerteschaltung.

Fig. 10 ein Diagramm zur Erläuterung eines weiteren Grundgedankens der Erfindung

Fig. 11 eine Ausführungsform der Vorrichtung zur Auswertung nach den Prinzipien der Fig. 10.

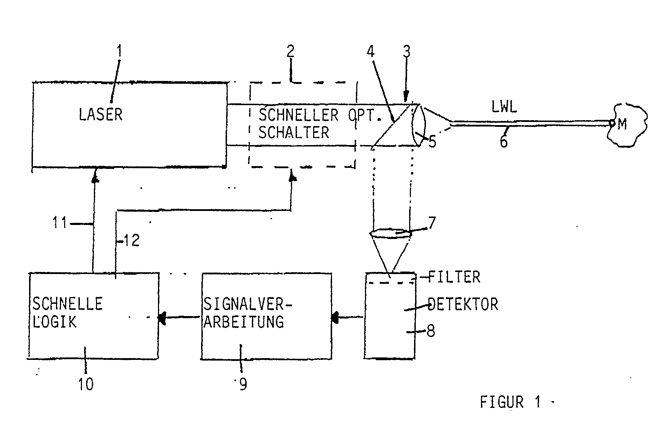

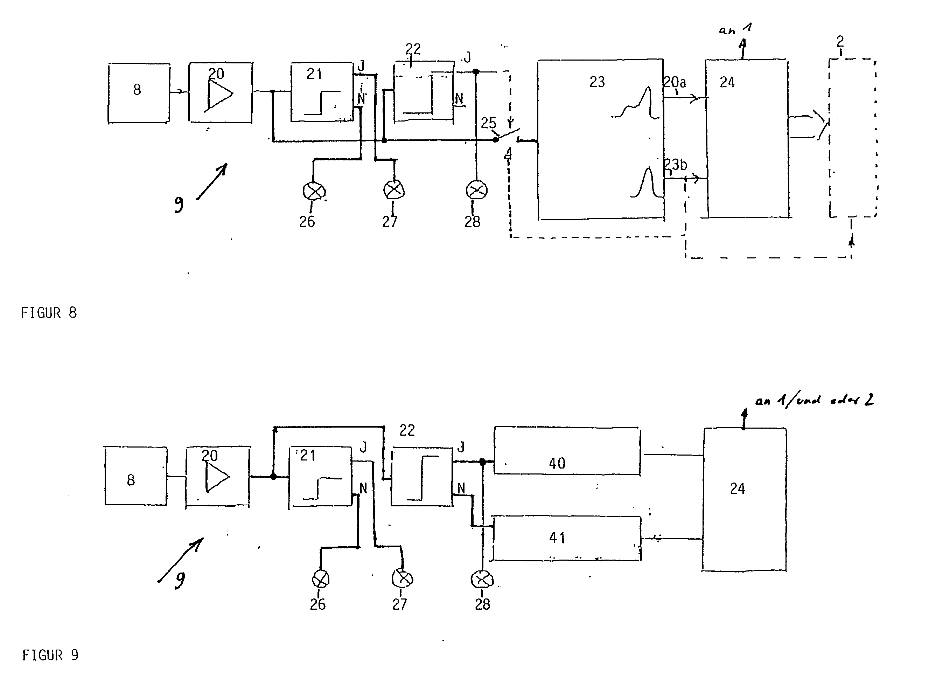

Die in Fig. 1 dargestellte Ausführungsform einer Vorrichtung weist einen Laser (1) auf, dessen Ausgangsimpulse über einen eine hohe Schaltgeschwindigkeit aufweisenden optischen Schalter (2) einer Laseroptik (3) zugeführt werden, deren Ausgang über einen Lichtwellenleiter (6) auf das zu bearbeitende Material (M) gerichtet wird. Bei der dargestellten Ausführungsform weist die Laseroptik (3) beispielsweise einen teildurchlässigen Spiegel (4) sowie eine erste Linse (5) zur Fokussierung des Laserlichtimpulses auf den Lichtwellenleiter (6) auf. Von dem zu bearbeitenden Material (M) reemittiertes, zurückgestreutes oder zurückreflektiertes Licht gelangt über den Lichtwellenleiter (6), die Linse (5) und den teildurchlässigen Spiegel (4) auf eine weitere Linse (7), die dieses Licht auf eine Detektoranordnung fokussiert. Das Ausgangssignal wird einer anhand der Fig. 8 und 9 noch näher zu beschreibenden Auswerteschaltung zugeführt, die über Logikschaltungen (10) den optischen Schalter (2) über eine Ausgangsleitung (12) steuert. Wie dies durch eine weitere Ausgangsleitung (11) der Logikschaltung (10) angedeutet ist, kann diese auch den Laser (1) selbst steuern, wobei gegebenfalls der optische Schalter (2) entfallen kann.

Bei Auftreffen eines Laserimpulses hoher Energie auf das zu bearbeitende Material (M) wird in unmittelbarer Nähe dieses zu bearbeitenden Materials ein dielektrischer Durchbruch erzeugt, der eine Schockwelle zur Bearbeitung des Materials auslöst.

Dieses Material kann beispielsweise ein menschlicher Stein, beispielsweise ein Harn- oder Gallenstein sein, der in umgebendes Gewebe eingebettet ist. In diesem Fall bewirkt die Schockwelle eine Steinzertrümmerung. Die entstehende Plasmablase bewirkt durch die hohe Temperatur des Plasmas und die entstehende Druckwelle eine Materialabsprengung, Materialabtragung oder Aufspaltung am Zielort. Hierbei muß jedoch in vielen Fällen, insbesondere bei dem betrachteten Fall von menschlichen Steinen darauf geachtet werden, daß der am Ende des Lichtwellenleiters (6) austretende Laserimpuls lediglich auf das zu bearbeitende Material (M), nicht jedoch auf das umgebende Gewebe auftritt, damit dieses nicht zerstört wird.

Die Detektoranordnung (8) ermöglicht in Verbindung mit der Auswerteschaltung (9) eine sehr schnelle Ermittlung, ob zu bearbeitendes Material am Ende des Lichtwellenleiters (6) angeordnet ist, oder ob dieses Ende des Lichtwellenleiters auf Material gerichtet ist, das nicht mit dem Laserimpuls, zumindestens nicht mit einem Laserimpuls voller Leistung beaufschlagt werden darf.

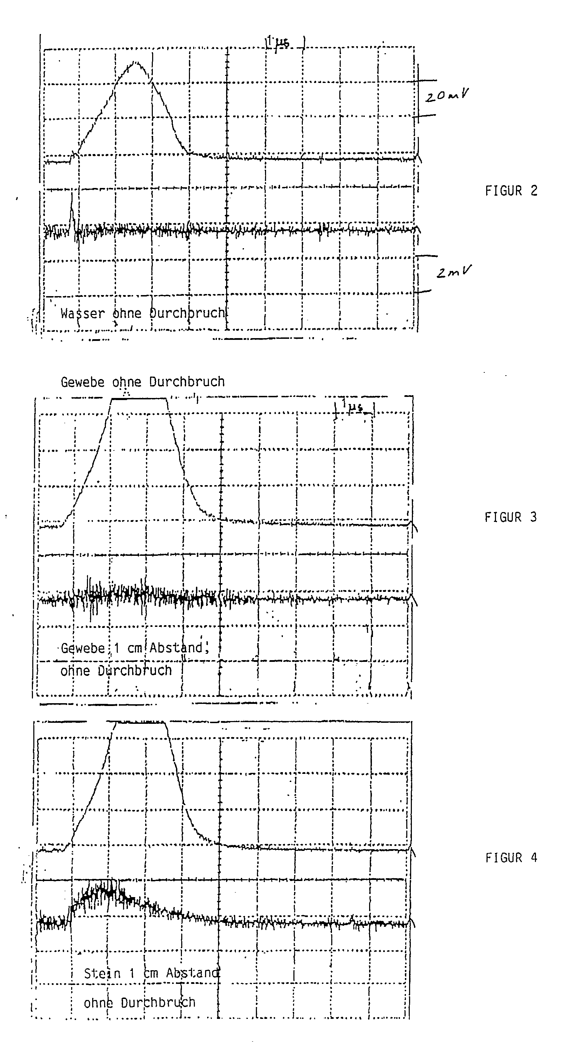

Zu diesem Zweck wertet die Auswerteschaltung den zeitlichen Funktionsverlauf des auf die Detektoranordnung einfallenden Lichtes aus, wobei diese Auswertung auf Grundgedanken beruht, die im folgenden anhand der Diagramme nach den Fig. 2-6 näher erläutert werden.

Im oberen Teil der Diagramme ist jeweils der Laserimpuls selbst dargestellt, während im unteren Teil das Ausgangssignal der Detektoranordnung dargestellt ist, wobei die Abszisse den Zeitverlauf und die Ordinate die Amplitude der jeweiligen Impulse darstellt.

Die Fig. 2-4 zeigen den Fall der Aussendung von Laserimpulsen geringer Leistung, die nachfolgend als Meßimpulse bezeichnet werden.

In Fig. 2 ist der Fall dargestellt, in dem das Ende des Lichtwellenleiters (6) auf Wasser gerichtet ist. Es ist zu erkennen, daß hierbei lediglich fast im Rauschbereich liegende Ausgangssignale der Detektoranordnung auftreten.

In Fig. 3 ist der Fall dargestellt, in dem das Ende des Lichtwellenleiters (6) in einem Abstand von einem Zentimeter von menschlichem Gewebematerial angeordnet ist. In diesem Fall ist zu erkennen, daß das Ausgangssignal der Detektoranordnung (8) wesentlich höhere Amplituden erreicht, als im Fall der Fig. 2.

In Fig. 4 ist schließlich der Fall dargestellt, in dem das Ende des Lichtwellenleiters in einem Abstand von einem Zentimeter vor einem zu bearbeitenden menschlichen Stein angeordnet ist, der zertrümmert werden soll. Es ist aus einem Vergleich der Fig. 2-4 deutlich zu erkennen, daß bei Aussendung des Meßimpulses bei Annäherung an einen Stein eine wesentlich höhere Amplitude des Ausgangssignals der Detektoranordnung auftritt, die zur Feststellung der Tatsache verwendet werden kann, daß das Ende des Lichtwellenleiters benachbart zu einem Stein angeordnet ist.

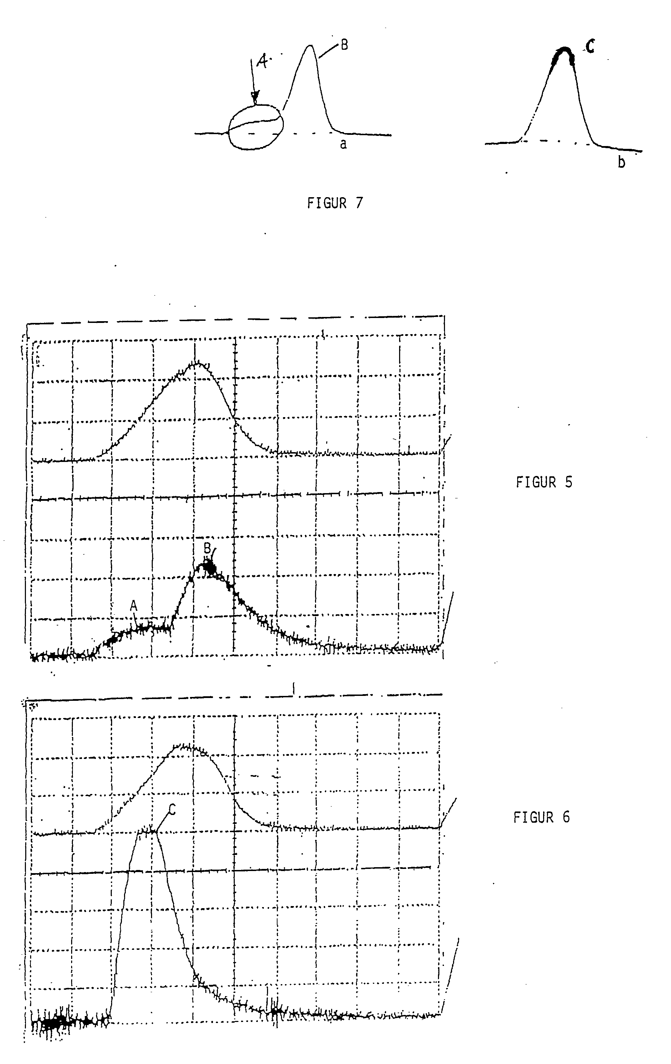

Diese Verhältnisse kehren sich in gewisser Weise bei Aussendung eines Bearbeitungsimpulses um, wie dies aus den Fig. 5 und 6 zu erkennen ist. Fig. 5 zeigt den Fall, bei dem die Energie des Laserimpulses so hoch ist, daß am Ende des Lichtwellenleiters (6) ein dielektrischer Durchbruch erfolgt. In diesem Fall tritt bei Auftreffen des Laserimpulses auf den Stein zunächst ein relativ flacher Anstieg (A) gemäß Fig. 5 auf, auf den eine höhere Impulsspitze am Ausgang der Detektoranordnung (8) folgt. Wenn jedoch der Durchbruch in Gewebematerial erfolgt, so tritt unmittelbar eine einzige relativ hohe Impulsspitze (C) nach Fig. 6 auf.

Die Fig. 7a und 7b zeigen diesen zeitlichen Funktionsverlauf in idealisierter Darstellung, wobei die Fig. 7a der Fig. 5 entspricht, während die Fig. 7b der Fig. 6 entspricht. Es ist zu erkennen, daß bei einem dielektrischen Durchbruch an einem Stein gemäß Fig. 7a ebenfalls wieder zunächst ein relativ flacher Anstieg (A) des Ausgangssignals der Detektoranordnung auftritt, auf den eine eindeutige Impulsspitze (B) folgt. Bei Auftreten des dielektrischen Durchbruches in Gewebe gemäß Fig. 7b tritt lediglich eine einzige Impulsspitze (C) auf.

Aufgrund dieser Erkenntnis des zeitlichen Funktionsverlaufes des auf die Detektoranordnung nach Fig. 1 auftreffenden Lichtes ist es möglich, mit geringem Aufwand Auswerteschaltungen aufzubauen, die eine eindeutige Erkennung ermöglichen, ob das Ende des Lichtwellenleiters auf ein das zu bearbeitende Material umgebendes Material oder auf das zu bearbeitende Material selbst gerichtet ist.

Ausführungsbeispiele derartiger Auswerteschaltungen sind in den Fig. 8 und 9 dargestellt.

Beiden Ausführungsformen ist gemeinsam, daß der Detektoranordnung (8) ein Signalverstärker (20) nachgeschaltet ist, dessen Ausgangssignal den Eingängen eines ersten Schwellwertdetektors (21) und eines zweiten Schwellwertdetektors (22) zugeführt wird.

Beiden Schwellwertdetektoren ist gemeinsam, daß sie über zwei Ausgänge verfügen, die mit J bzw. N bezeichnet sind. An dem Ausgang (J) der Schwellwertdetektoren (21,22) erscheint lediglich dann ein Ausgangssigmal, wenn die jeweilige Schwelle des Schwellwertdetektors überschritten wird. In allen anderen Fällen erscheint am Ausgang (N) dieser Schwellwertdetektoren ein Ausgangssignal.

Weiterhin ist bei beiden Ausführungsformen nach den Fig. 8 und 9 die Schwelle des zweiten Schwellwertdetektors (22) höher als die des ersten Schwellwertdetektors (21).

Der 'N'-Ausgang bzw. der 'J'-Ausgang des Schwellwertdetektors (21) ist jedem Fall mit Anzeigeeinrichtungen (26 bzw. 27) verbunden, die in noch zu beschreibender Weise beim Suchvorgang bzw. bei der Feststellung von Gewebe am Ende des Lichtwellenleiters (8) aktiviert werden.

Der mit 'J' bezeichnete Ausgang des zweiten Schwellwertdetektors ist weiterhin mit einer Anzeigeeinrichtung (28) verbunden, die zusätzlich anzeigt, daß sich das Ende des Lichtwellenleiters benachbart zu einem zu bearbeitendem Material, beispielsweise dem Stein befindet.

Bei der Ausführungsform nach Fig. 8 betätigt der mit 'J' bezeichnete Ausgang des Schwellwertschalters (22) weiterhin einen Schalter (25), der selbstverständlich ein elektronischer Schalter sein kann. Dieser Schalter verbindet das Ausgangssignal des Signalverstärkers (20) mit dem Eingang eines Kurvenformdiskriminators (23), der den zeitlichen Funktionsverlauf des Ausgangssignals der Detektoranordnung (8) feststellt. Beim Schließen des Schalters (25) liefert der Kurvenformdiskriminator (23) über die Ausgangsleitung (20a) ein Signal an die Lagersteuerschaltung (24), die den Laser (1) zur Abgabe eines Bear beitungsimpulses ansteuert. Wenn der Kurvenformdiskriminator dann den für zu bearbeitendes Material typischen zeitlichen Funktionsverlauf gemäß Fig. 7a oder Fig. 5 feststellt, so steuert er über die Lasersteuerschaltung (24) den Laser fortlaufend derart an, daß dieser Bearbeitungsimpulse mit einer Leistung abgibt, die für einen dielektrischen Durchbruch ausreicht. Die Abgabe von Bearbeitungsimpulsen hoher Leistung wird solange fortgesetzt, bis der Kurvenformdiskriminator die Kurvenform nach Fig. 6 bzw. 7b feststellt, worauf über eine zweite Ausgangsleitung (23b) die Lasersteuerung (24) so angesteuert wird, daß die Leistung unmittelbar verringert wird, wobei gegebenenfalls auch der optische Schalter (2) angesteuert wird, um die Weiterleitung des Ausgangsimpulses des Lasers an den Lichtwellenleiter unmittelbar zu unterbrechen, sodaß der Laserausgangsimpuls, der am Ende des Lichtwellenleiters auftritt, verkürzt wird. Ohne Verwendung des optischen Schalters wird bei kürzeren Laserimpulsen der nächste Laserimpuls in seiner Leistungsdichte reduziert (Meßimpuls). Das Ausgangssignal am zweiten Ausgang (23b) des Kurvenformdiskriminators wird weiterhin dazu verwendet, den Schalter (25) in den offenen Zustand zurückzusetzen. Es werden dann lediglich Laserimpulse geringerer Leistung ausgesandt, die zum Suchen von weiterem zu bearbeitenden Material verwendet werden, worauf wieder bei Feststellung von zu bearbeitendem Material in der eingangs genannten Weise ein Schließen des Schalters (25) erfolgt und sich der Vorgang wiederholt.

Die Ausführungsform nach Fig. 9 ist insbesondere für ein Verfahren geeignet, bei dem jeweils abwechselnd ein Meßimpuls geringer Leistung und ein Bearbeitungsimpuls höherer Leistung aufeinanderfolgen, wobei jedoch der Bearbeitungsimpuls nur dann ausgelöst wird, wenn durch den vorhergehenden Meßimpuls zu bearbeitendes Material am Ende des Lichtwellenleiters (6) festgestellt wurde.

Zu diesem Zweck ist der mit 'J' bezeichnete Ausgang des zweiten Schwellwertschalters (22) mit einer ersten Triggerschaltung (40) verbunden, die die Lasersteuerung (24) derart ansteuert, daß der Laser einen Bearbeitungsimpuls abgibt.

Sofern der mit 'N' bezeichnete Ausgang des Schwellwertschalters (22) aktiviert ist, wird lediglich ein schwacher Meßimpuls über eine zweite Triggerschaltung (41) ausgelöst.

Selbstverständlich kann weiterhin der ersten Triggerschaltung (40) ein Kurvenformdiskriminator (23) gemäß Fig. 8 nachgeschaltet sein, der eine Auswertung dieser Kurvenform ermöglicht.

Dieser Kurvenformdiskriminator kann beispielsweise eine Fouriertransformation ausführen, und er könnte durch einen schnellen Analog-/ Digitalwandler und einen Schieberegister einschließenden Vergleicher gebildet sein. Weitere Ausführungsformen derartiger Kurvenformdiskriminatoren sind dem Fachmann gut bekannt.

An dem der Laseroptik benachbarten Ende des Lichtwellenleiters (6) oder an einer anderen geeigneten Stelle kann weiterhin ein akustischer Detektor angeordnet sein, der die den dielektrischen Durchbruch begleitenden Schallsignale erfaßt und für eine weitere, nicht dargestellte Auswertung bereitstellt.

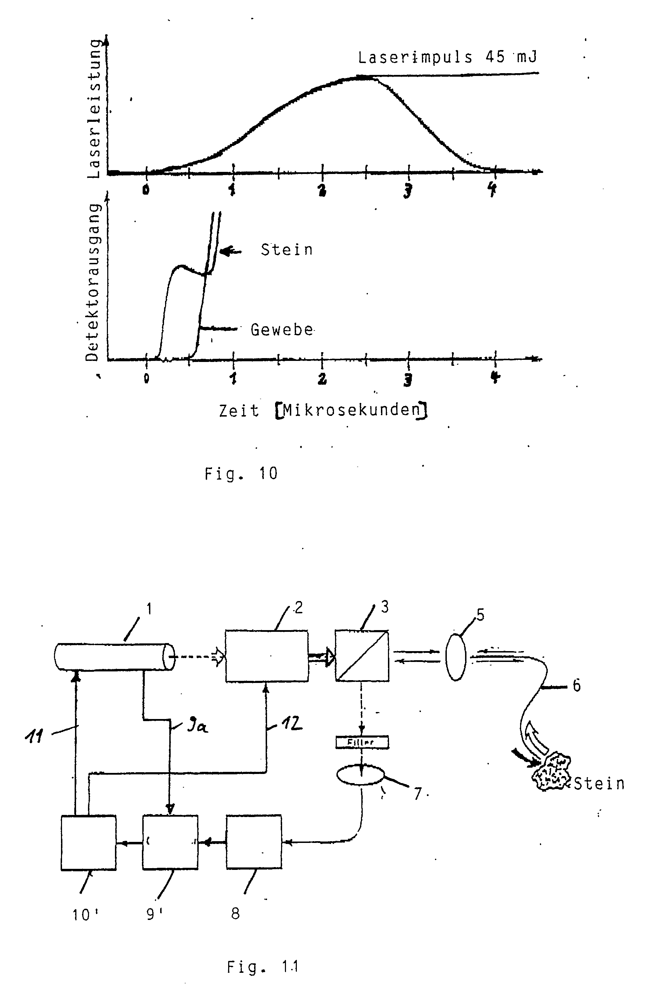

Wie ein Vergleich der Figuren 5 und 6 zeigt, ergibt sich im Fall des in Fig. 5 dargestellten Auftreffens des Laserimpulses auf relativ hartes Material bereits kurz nach dem Auslösen des in der oberen Hälfte dieser Figur dargestellten Laserimpulses ein Anstieg des Ausgangssignals an der Stelle A. Demgegenüber erfolgt der Anstieg des Ausgangssignals der Detektoranordnung gemäß Fig. 6 bei Auftreffen auf relativ weiches Material wesentlich später, und diese beiden Diagramme nach den Figuren 5 und 6 sind zur Verdeutlichung in Fig. 10 kombiniert dargestellt. Aus dem Diagramm der Figur 10 ist zu erkennen, daß bei Auftreffen auf einen Stein das Ausgangssignal der Detektoranordnung bereits nach etwa 100 - 150 Nanosekunden ansteigt, während bei Auftreffen auf Gewebe dieser Anstieg erst etwa 500 Nanosekunden nach Auslösen des Laserimpulses erfolgt. Zu diesem Zeitpunkt hat der Laserimpuls seine volle Leistung noch nicht erreicht, so daß es über die Leitung (11) und/oder die Leitung (12) mit Hilfe des schnellen optischen Schalters (2) nach den Fig.1 und 11 möglich ist, den am Ende des Lichtwellenleiters (6) austretenden Laserimpuls vor Erreichen der maximalen Energie zu beenden.

Die erfindungsgemäße Feststellung der in Fig. 10 dargestellten Verhältnisse ermöglicht eine relativ einfache Ausgestaltung der Auswerteschaltung in Form eines Vergleichers (9') nach Fig. 11, der beispielsweise ein Zeitglied enthält, das im vorstehenden Fall etwa 200 Nanosekunden nach dem Auslösen des Laserimpulses das Ausgangssignal des Detektors abtastet, um festzustellen, ob das Ausgangssignal des Detektors einen vorgegebenen Schwellwert erreicht oder überschritten hat. Wenn dies nicht der Fall ist, gibt der Vergleicher (9') ein Ausgangssignal an eine Steuerschaltung (10') ab, die den Laser (1) abschaltet und/oder den schnellen optischen Schalter (2) sperrt, sodaß der in den Lichtwellenleiter eintretende Laserimpuls vor Erreichen seiner maximalen Energie beendet wird.

Die Ausführungsform der Vorrichtung nach Fig. 11 ergibt damit bei vereinfachtem Aufbau eine sehr wirkungsvolle und zuverlässige Abschaltung des Laserimpulses, falls dieser versehentlich auf nicht zu bearbeitendes Material auftrifft.

**Patentansprüche**

1. Verfahren zur Materialbearbeitung mit Hilfe eines Lasers (1), unter Erkennung des zu bearbeitenden Materials (M), bei dem das Laserlicht über eine Laseroptik (3) auf das Material gerichtet und das von dem Material reemittierte, zurückreflektierte oder zurückgestreute Licht über die Laseroptik (3) auf eine Detektoranordnung (8) geleitet wird, deren Ausgangssignal einer Auswerteschaltung (9) zur Steuerung der Laserleistung oder -energie zugeführt wird,

dadurch **gekennzeichnet,** daß für die Steuerung des Lasers und/oder eines im Ausgangsstrahlengang des Lasers angeordneten optischen Schalters (2) der zeitliche Funktionsverlauf des auf die Detektoranordnung einfallenden Lichtes bezogen auf den zeitlichen Verlauf des Laserimpulses ausgewertet wird, daß bis zum Erreichen einer gewünschten Relativposition zwischen der Laseroptik und dem zu bearbeitenden Material der Laser mit verringerter Leistung betrieben wird, daß nach dem Erreichen der gewünschten Relativposition zwischen der Laseroptik und dem zu bearbeitenden Material zunächst ein Laser-Meßimpuls geringerer Leistung ausgesandt wird, der zur Überprüfung der Art und/oder des Zustandes des vor der Laseroptik befindlichen Materials dient, und daß bei Feststellung des richtigen Materials und/oder Materialzustandes nachfolgend ein Laserimpuls erhöhter Leistung zur Materialbearbeitung ausgesandt wird, worauf wiederum ein Meßimpuls folgt.

2. Verfahren zur Materialbearbeitung mit Hilfe eines Lasers (1), unter Erkennung des zu bearbeitenden Materials (M), bei dem das Laserlicht über eine Laseroptik (3) auf das Material gerichtet und das von dem Material reemittierte, zurückreflektierte oder zurückgestreute Licht über die Laseroptik (3) auf eine Detektoranordnung (8) geleitet wird, deren Ausgangssignal einer Auswerteschaltung (9) zur Steuerung der Laserleistung oder -energie zugeführt wird,

dadurch **gekennzeichnet,** daß für die Steuerung des Lasers und/oder eines im Ausgangsstrahlengang des Lasers angeordneten optischen Schalters der zeitliche Funktionsverlauf des auf die Detektoranordnung einfallenden Lichtes bezogen auf den zeitlichen Verlauf des Laserimpulses ausgewertet wird, und daß der dem zu bearbeitenden Material zugeführte Laserimpuls innerhalb einer vorgegebenen Zeit nach seiner Auslösung unterbrochen wird, wenn bis zu dieser Zeit ein vorgegebenes Meßkriterium des Ausgangssignals der Detektoranordnung nicht festgstellt wurde.

3. Verfahren nach Anspruch 2, dadurch **gekennzeichnet,** daß das vorgegebene Meßkriterium eine oberhalb eines Schwellwertes liegende Amplitude des Ausgangssignals der Detektoranordnung ist.

4. Verfahren nach einem der vorhergehenden Ansprüche,
dadurch **gekennzeichnet,** daß der zeitliche Funktionsverlauf des auf die Detektoranordnung einfallenden Lichts gemessen und zur Feststellung der Tatsache herangezogen wird, ob die Laseroptik auf zu bearbeitendes Material ausgerichtet ist.

5. Verfahren nach Anspruch 4,
dadurch **gekennzeichnet,** daß bei Aussendung des Meßimpulses bei Ausrichtung auf zu bearbeitendes Material eine höhere Rückstreuung als bei Ausrichtung auf das das zu bearbeitende Material einbettendes Material auftritt, während bei Aussendung des Bearbeitungsimpulses bei Ausrichtung auf das zu bearbeitende Material eine andere Zeitfunktion des auf die Detektoranordnung einfallenden Lichtes als bei Ausrichtung auf das einbettende Material festgestellt wird, und daß diese Kriterien zur Steuerung des Lasers und/oder des optischen Schalters verwendet werden.

6. Verfahren nach einem der vorhergehenden Ansprüche,
dadurch **gekennzeichnet,** daß zusätzlich das beim dielektrischen Durchbruch entstehende akustische Signal erfaßt und ausgewertet wird.

7. Vorrichtung zur Materialbearbeitung mit Hilfe eines Lasers (1), dem eine Laseroptik (3) nachgeschaltet ist, die die Laserimpulse auf das zu bearbeitende Material (M) lenkt und von diesem reemittiertes, zurückreflektiertes oder zurückgestreutes Licht auf eine Detektoranordnung umlenkt, der eine Auswerteschaltung (9) zur Steuerung der Energie und/oder der Dauer des von dem Laser abgegebenen Impulses oder der Impulse nachgeschaltet ist,

dadurch **gekennzeichnet,** daß die Auswerteschaltung (9) den zeitlichen Funktionsverlauf des auf die Detektoranordnung (8) einfallenden Lichtes bezogen auf den zeitlichen Verlauf des Laserimpulses feststellt, daß die Auswerteschaltung (9;9') in Abhängigkeit von dem zeitlichen Verlauf des auf die Detektoranordnung (8) einfallenden Lichtes den Laser (1) und/oder einen zwischen dem Laser (1) und der Laseroptik (3) eingeschalteten optischen Schalter (2) steuert, daß die Auswerteschaltung (9) einen ersten und einen zweiten Schwellwertdetektor (21,22) einschließt, deren Eingänge mit dem Ausgang der Detektoranordnung (8) verbunden sind, daß die Schwelle (Us2) des zweiten Schwellwertdetektors (22) größer als die Schwelle (Us1) des ersten Schwellwertdetektors (21) ist, und daß der bei Überschreiten der Schwelle (Us2) des zweiten Schwellwertdetektors (22) aktive Ausgang die Steuerung des Lasers (1) und/oder des optischen Schalters (2) bewirkt (Fig. 8, 9).

8. Vorrichtung nach Anspruch 7,
dadurch **gekennzeichnet,** daß der bei Überschreiten der zweiten Schwelle (Us2) des zweiten Schwellwertdetektors (22) aktive Ausgang eine Schalteinrichtung (25) steuert, die den Ausgang der Detektoranordnung (8) mit dem Eingang eines Kurvenformdiskriminators zur Analyse der zeitlichen Funktion des Ausgangssignals der Detektoranordnung (8) verbindet, und daß der Kurvenformdiskriminator (23) in Abhängigkeit von dem zeitlichen Funktionsverlauf des Ausgangssignals der Detektoranordnung (8) eine Laserschaltung (24) ansteuert, die die Abgabe eines Laserimpulses mit größerer und kleinerer Leistung und /oder die Durchlässigkeit des optischen Schalter (2) steuert.

9. Vorrichtung nach Anspruch 7,
dadurch **gekennzeichnet,** daß der bei Überschreiten der Schwelle (Us2) des zweiten Schwellwertdetektors (22) aktive Ausgang mit dem Eingang einer ersten Triggerschaltung (40) verbunden ist, die über eine Laser-Steuerschaltung die Auslösung eines Bearbeitungsimpulses bewirkt, während der das fehlende Überschreiten der Schwelle darstellende Ausgang des zweiten Schwellwertschalters (22) mit einer zweiten Triggerschaltung (41) verbunden ist, die über die Laser-Steuerschaltung (24) die Auslösung eines Meßimpulses bewirkt.

10. Vorrichtung zur Materialbearbeitung mit Hilfe eines Lasers (1), dem eine Laseroptik (3) nachgeschaltet ist, die die Laserimpulse auf das zu bearbeitende Material (M) lenkt und von diesem reemittiertes, zurückreflektiertes oder zurückgestreutes Licht auf eine Detektoranordnung umlenkt, der eine Auswerteschaltung (9) zur Steuerung der Energie und/oder der Dauer des von dem Laser angegebenen Impulses oder der Impulse nachgeschaltet ist,
dadurch **gekennzeichnet,** daß die Auswerteschaltung (9) den zeitlichen Funktionsverlauf des auf die Detektoranordnung (8) einfallenden Lichts bezogen auf den zeitlichen Verlauf des Laserimpulses feststellt, daß die Auswerteschaltung (9,9') in Abhängigkeit von dem zeitlichen Verlauf des auf die Detektoranordnung (8) eifallenden Lichtes den Laser (1) und/oder einen zwischen dem Laser (1) und der Laseroptik (3) eingeschalteten optischen Schalter (2) steuert, daß die Auswerteschaltung (9') einen Vergleicher einschließt, der ein Ausgangssignal liefert, wenn das Ausgangssignal der Detektoranordnung (8) nicht innerhalb einer vorgegebenen Zeit nach Auslösen eines Laserimpulses einen Schwellwert erreicht hat (Fig. 11).

11. Vorrichtung nach Anspruch 10,
dadurch **gekennzeichnet,** daß das Ausgangssignal des Vergleichers den Laser und/oder eine im Strahlengang des LAsers (1) angeordnete optische Sperrzelle (2) steuert.

12. Vorrichtung nach einem der Ansprüche 7 bis 11,
dadurch **gekennzeichnet,** daß die Laseroptik (3) einen teildurchlässigen Spiegel (4) einschließt, der vor dem mit dem Laserimpuls beaufschlagten Material (M) reemittiertes, zurückgestreutes oder zurückreflektiertes Licht auf die Detektoranordnung (8) umlenkt.

13. Vorrichtung nach einem der Ansprüche 7 bis 11,
dadurch **gekennzeichnet,** daß die Laseroptik einen ersten Lichtwellenleiter, der die Ausgangsimpulse auf das zu bearbeitende Material leitet, und einen zweiten, zum ersten im wesentlichen parallelen Lichtwellenleiter einschließt, der das von dem Material reemittierte, reflektierte oder zurückdestreute Licht auf die Detektoranordnung (8) lenkt.

14. Vorrichtung nach einem der Ansprüche 7 bis 13,
dadurch **gekennzeichnet,** daß die Detektoranordnung (8) mehrere Detektoren umfaßt, die auf unterschiedliche spektrale Bereiche des von dem Material (M) reemittierten, zurückgestreuten oder zurückreflektierten Lichtes ansprechen.

**Claims**

1. Method of material treatment with the aid of a laser (1) with detection of the material (M) to be treated, in which the laser light is directed via laser optics (3) onto the material and the light re-emitted, reflected back or scattered back from the material is directed via the laser optics (3) onto a detector arrangement (8), the output signal of which is fed to an evaluation circuit (9) for controlling the laser power or energy, characterised in that, for controlling the laser and/or an optical switch (2) arranged in the output ray path of the laser, the variation as a function of time of the light incident on the detector arrangement is evaluated with reference to the variation over time of the laser pulse, in that the laser is operated at reduced power until a desired relative position between the laser optics and the material to be treated is reached, in that, after reaching the desired relative position between the laser optics and the material to be treated, initially a laser measuring pulse of lower power is emitted, which serves for checking the nature and/or condition of the material located in front of the laser optics, and in that, on establishing the correct material and/or material condition, from then on a laser pulse of increased power is emitted for material treatment, after which again a measuring pulse follows.

2. Method of material treatment with the aid of a laser (1) with detection of the material (M) to be treated, in which the laser light is directed via laser optics (3) onto the material and the light re-emitted, reflected back or scattered back from the material is directed via the laser optics (3) onto a detector arrangement (8), the output signal of which is fed to an evaluation circuit (9) for controlling the laser power or laser energy, characterised in that, for controlling the laser and/or an optical switch arranged in the output ray path of the laser, the variation as a function of time of the light incident on the detector arrangement is evaluated with reference to the variation over time of the laser pulse, and in that the laser pulse fed to the material to be treated is interrupted within a predetermined time, if by this time a predetermined measuring criterion of the output signal of the detector arrangement has not been established.

3. Method according to Claim 2, characterised in that the predetermined measuring criterion is an amplitude of the output signal of the detector arrangement lying above a threshold value.

4. Method according to one of the preceding claims, characterised in that the variation as a function of time of the light incident on the detector arrangement is measured and used for establishing the fact whether the laser optics are aligned with material to be treated.

5. Method according to Claim 4, characterised in that a higher backscatter occurs when the measuring pulse is emitted in the case of alignment with material to be treated than in the case of alignment with the material embedding the material to be treated, whereas a different time function of the light incident on the detector arrangement is established in the case of alignment with the material to be treated than in the case of alignment with the embedding material, and in that these criteria are used for controlling the laser and/or the optical switch.

6. Method according to one of the preceding claims, characterised in that, in addition, the acoustic signal produced upon the dielectric breakdown is detected and evaluated.

7. Apparatus for material treatment with the aid of a laser (1), which is followed by laser optics (3), which direct the laser pulses to the material (M) to be treated and deflect to a detector arrangement light re-emitted, reflected back or scattered back, which detector arrangement is followed by an evaluation circuit (9) for controlling the energy and/or the duration of the pulse or pulses emitted by the laser, characterised in that the evaluation circuit (9) establishes the variation as a function of time of the light incident on the detector arrangement (8) with reference to the variation over time of the laser pulse, in that the evaluation circuit (9; 9') controls the laser (1) and/or an optical switch (2) connected in between the laser (1) and the laser optics (3) in dependence on the variation over time of the light incident on the detector arrangement (8), in that the evaluation circuit (9) includes a first and a second threshold detector (21, 22), the inputs of which are connected to the output of the detector arrangement (8), in that the threshold (Us2) of the second threshold detector (22) is greater than the threshold (Us1) of the first threshold detector (21) and in that the output active when the threshold (Us2) of the second threshold detector (22) is exceeded effects the controlling of the laser (1) and/or the optical switch (2) (Figs. 8, 9).

8. Apparatus according to Claim 7, characterised in that the output active when the second threshold (Us2) of the second threshold detector (22) is exceeded controls a switching device (25), which connects the output of the detector arrangement (8) to the input of a curve shape discriminator for analysis of the function over time of the output signal of the detector arrangement (8), and in that the curve shape discriminator (23) activates a laser circuit (24) in dependence on the variation as a function of time of the output signal of the detector arrangement (8), which laser circuit controls the emission of a laser pulse with greater and lesser power and/or the transmission of the optical switch (2).

9. Apparatus according to Claim 7, characterised in that the output active when the threshold (Us2) of the second threshold detector (22) is exceeded is connected to the input of the first trigger circuit (40), which effects the triggering of a treatment pulse by means of a laser control circuit, whereas the output of the second threshold switch (sic) (22), representing not exceeding of the threshold, is connected to a second trigger circuit (41), which effects the triggering of a measuring pulse by means of the laser control circuit (24).

10. Apparatus for material treatment with the aid of a laser (1), which is followed by laser optics (3), which direct the laser pulses to the material (M) to be treated and deflect to a detector arrangement light re-emitted, reflected back or scattered back, which detector arrangement is followed by an evaluation circuit (9) for controlling the energy and/or the duration of the pulse or pulses emitted by the laser, characterised in that the evaluation circuit (9) establishes the variation as a function of time of the light incident on the detector arrangement (8) with respect to the variation over time of the laser pulse, in that the evaluation circuit (9, 9') controls the laser (1) and/or an optical switch (2) connected in between the laser (1) and the laser optics (3) in dependence on the variation over time of the light incident on the detector arrangement (8), in that the evaluation circuit (9') includes a comparator, which supplies an output signal if the output signal of the detector arrangement (8) has not reached a threshold value within a predetermined time after triggering of a laser pulse (Fig. 11).

11. Apparatus according to Claim 10, characterised in that the output signal of the comparator controls the laser and/or an optical barrier cell (2) arranged in the ray path of the laser (1).

12. Apparatus according to one of Claims 7 to 11, characterised in that the laser optics (3) include a semi-transparent mirror (4), which deflects onto the detector arrangement (8) light re-emitted, scattered back or reflected back from the material (M) impinged with the laser pulse.

13. Apparatus according to one of Claims 7 to 11, characterised in that the laser optics include a first optical waveguide, which directs the output pulses to the material to be treated, and a second optical waveguide, essentially parallel to the first, which guides onto the detector arrangement (8) the light re-emitted, reflected or scattered back from the material.

14. Apparatus according to one of Claims 7 to 13, characterised in that the detector arrangement (8) comprises a plurality of detectors, which respond to different spectral ranges of the light re-emitted, scattered back or reflected back from the material (M).

**Revendications**

1. Procédé pour le traitement de matières à l'aide d'un laser (1), accompagné de la détection de la matière à traiter (M), dans lequel la lumière laser est dirigée sur la matière par l'intermédiaire d'une optique laser (3), et la lumière réémise, réfléchie ou diffusée en retour par la matière est dirigée par l'intermédiaire de l'optique laser (3) sur un dispositif détecteur (8) dont le signal de sortie est amené à un circuit d'évaluation (9) pour commander la puissance ou l'énergie du laser, caractérisé par le fait que, pour la commande du laser et/ou d'un commutateur optique (2) disposé sur le trajet des rayons de sortie du laser, on évalue l'allure de la courbe en fonction du temps de la lumière qui arrive sur le dispositif détecteur, rapportée au tracé dans le temps de l'impulsion laser, par le fait que l'on fait fonctionner le laser à une puissance réduite jusqu'à ce que l'on atteigne une position relative voulue entre l'optique laser et la matière à traiter, par le fait qu'après que l'on a atteint la position relative voulue entre l'optique laser et la matière à traiter, on émet tout d'abord une impulsion laser de mesure de faible puissance servant à vérifier la nature et/ou l'état de la matière qui se trouve devant l'optique laser, et par le fait que, lorsque l'on a établi que la matière et/ou l'état de la matière est correct, on émet ensuite une impulsion laser de puissance plus élevée pour le traitement de la matière, laquelle est suivie à nouveau par une impulsion de mesure.

2. Procédé pour le traitement de matières à l'aide d'un laser (1), accompagné de la détection de la matière à traiter (M), dans lequel la lumière laser est dirigée sur la matière par l'intermédiaire d'une optique laser (3), et la lumière réémise, réfléchie ou diffusée en retour par la matière est dirigée par l'intermédiaire de l'optique laser (3) sur un dispositif détecteur (8) dont le signal de sortie est amené à un circuit d'évaluation (9) pour commander la puissance ou l'énergie du laser, caractérisé par le fait que, pour la commande du laser et/ou d'un commutateur optique disposé sur le trajet des rayons de sortie du laser, on évalue l'allure de la courbe en fonction du temps de la lumière qui arrive sur le dispositif détecteur, rapportée au tracé dans le temps de l'impulsion laser, et par le fait que l'on interrompt l'impulsion laser amenée à la matière à traiter à l'expiration d'une durée prédéterminée après son déclenchement lorsqu'il n'a pas encore été satisfait à cet instant à un critère de mesure prédéterminé pour le signal de sortie du dispositif détecteur.

3. Procédé selon la revendication 2, caractérisé par le fait que le critère de mesure prédéterminé est constitué par une amplitude du signal de sortie du dispositif détecteur qui est située au-dessus d'une valeur de seuil.

4. Procédé selon l'une des revendications précédentes, caractérisé par le fait que l'on détermine l'allure de la courbe en fonction du temps de la lumière qui arrive sur le dispositif détecteur, et qu'on l'utilise pour déterminer si l'optique laser est dirigée sur la matière à traiter.

5. Procédé selon la revendication 4, caractérisé par le fait que, lors de l'émission de l'impulsion de mesure, il se produit une dispersion en retour plus importante lors de l'alignement sur la matière à traiter que lors de l'alignement sur la matière qui noie la matière à traiter, tandis que, lors de l'émission de l'impulsion de traitement, on constate, pour la lumière arrivant sur le dispositif détecteur, une courbe dans le temps qui est différente lors de l'alignement sur la matière à traiter et lors de l'alignement sur la matière qui la noie, et par le fait que ces critères sont utilisés pour la commande du laser et/ou du commutateur optique.

6. Procédé selon l'une des revendications précédentes, caractérisé par le fait qu'en supplément, on détecte et on exploite le signal acoustique produit lors du claquage diélectrique.

7. Dispositif pour le traitement de matières à l'aide d'un laser (1) en aval duquel est montée une optique laser (3) qui dirige les impulsions laser sur la matière à traiter (M) et qui dévie la lumière réémise, réfléchie ou diffusée en retour par celle-ci sur un dispositif détecteur en aval duquel est monté un circuit d'évaluation (9) pour commander l'énergie et/ou la durée de l'impulsion fournie par le laser ou des impulsions, caractérisé par le fait que le circuit d'évaluation (9) détermine l'allure de la courbe en fonction du temps de la lumière qui arrive sur le dispositif détecteur (8), rapportée au tracé dans le temps de l'impulsion laser, par le fait que le circuit d'évaluation (9 ; 9') commande le laser (1) et/ou un commutateur optique (2) interposé entre le laser (1) et l'optique laser (3), et ce, en fonction de la courbe en fonction du temps de la lumière qui arrive sur le dispositif détecteur (8), par le fait que le circuit d'évaluation (9) comporte un premier et un second détecteur de valeur de seuil (21, 22) dont les entrées sont reliées à la sortie du dispositif détecteur (8), par le fait que le seuil (Us2) du second détecteur de valeur de seuil (22) est supérieur au seuil (Us1) du premier détecteur de valeur de seuil (21), et par le fait que la sortie qui est active lors du dépassement vers le haut du seuil (Us2) du second détecteur de valeur de seuil (22) produit la commande du laser (1) et/ou du commutateur optique (2) (figures 8 et 9).

8. Dispositif selon la revendication 7, caractérisé par le fait que la sortie qui est active lors du dépassement vers le haut du second seuil (Us2) du second détecteur de valeur de seuil (22) commande un dispositif de commutation (25) qui relie la sortie du dispositif détecteur (8) à l'entrée d'un discriminateur de formes de courbes en vue de l'analyse de la courbe dans le temps du signal de sortie du dispositif détecteur (8), et par le fait que le discriminateur de formes de courbes (23) excite, en fonction de l'allure de la courbe dans le temps du signal de sortie du dispositif détecteur (8), un commutateur laser (24) qui commande l'émission d'une impulsion laser de plus grande ou de plus faible puissance et/ou la transparence du commutateur optique (2).

9. Dispositif selon la revendication 7, caractérisé par le fait que la sortie qui est active lors du dépassement vers le haut du seuil (Us2) du second détecteur de valeur de seuil (22) est reliée à l'entrée d'un premier circuit de déclenchement (40) qui produit le déclenchement d'une impulsion de traitement par l'intermédiaire d'un circuit de commande du laser, tandis que la sortie du second détecteur de valeur de seuil (22) représentant le dépassement du seuil vers le haut qui est absent est reliée à un second circuit de déclenchement (41) qui produit le déclenchement d'une impulsion de mesure par l'intermédiaire du commutateur de commande du laser (24).

10. Dispositif pour le traitement de matières à l'aide d'un laser (1) en aval duquel est montée une optique laser (3) qui dirige les impulsions laser sur la matière à traiter (M) et qui dévie la lumière réémise, réfléchie ou diffusée en retour par celle-ci sur un dispositif détecteur en aval duquel est monté un circuit d'évaluation (9) pour commander l'énergie et/ou la durée de l'impulsion fournie par le laser ou des impulsions, caractérisé par le fait que le circuit d'évaluation (9) détermine l'allure de la courbe en fonction du temps de la lumière qui arrive sur le dispositif détecteur (8), rapportée au tracé dans le temps de l'impulsion laser, par le fait que le circuit d'évaluation (9 ; 9') commande le laser (1) et/ou un commutateur optique (2) interposé entre le laser (1) et l'optique laser (3), et ce, en fonction de la courbe en fonction du temps de la lumière qui arrive sur le dispositif détecteur (8), et par le fait que le circuit d'évaluation (9') comprend un comparateur qui fournit un signal de sortie lorsque le signal de sortie du dispositif détecteur (8) n'a pas atteint une valeur de seuil à l'intérieur d'une durée prédéterminée après le déclenchement d'une impulsion laser (figure 11).

11. Dispositif selon la revendication 10, caractérisé par le fait que le signal de sortie du comparateur commande le laser et/ou une cellule de blocage optique (2) disposée sur le trajet des rayons du laser (1).

12. Dispositif selon l'une des revendications 7 à 11, caractérisé par le fait que l'optique laser (3) comprend un miroir semi-transparent (4) qui dévie sur le dispositif détecteur (8) la lumière réémise, réfléchie ou diffusée en retour par la matière (M) frappée par l'impulsion laser.

13. Dispositif selon l'une des revendications 7 à 11, caractérisé par le fait que l'optique laser comprend un premier guide d'ondes lumineuses qui dirige les impulsions de sortie sur la matière à traiter et un second guide d'ondes lumineuses qui est pour l'essentiel parallèle au premier et qui amène au dispositif détecteur (8) la lumière réémise, réfléchie ou diffusée en retour par la matière.

14. Dispositif selon l'une des revendications 7 à 13, caractérisé par le fait que le dispositif détecteur (8), comprend plusieurs détecteurs qui répondent sur des zones spectrales différentes de la lumière réémise, réfléchie ou diffusée en retour par la matière (M).

FIGUR 1

FIGUR 2

Wasser ohne Durchbruch

Gewebe ohne Durchbruch

FIGUR 3

Gewebe 1 cm Abstand, ohne Durchbruch

FIGUR 4

Stein 1 cm Abstand ohne Durchbruch

FIGUR 7

FIGUR 5

FIGUR 6

14

FIGUR 8

FIGUR 9

Fig. 10

Fig. 11